# EUROPEAN PATENT SPECIFICATION

(11) **EP 2 655 651 B1**
(45) Date of publication and mention of the grant of the patent: **21.11.2018**
(21) Application number: 11850890.2
(22) Date of filing: 21.12.2011
(51) Int. Cl.: C12P 21/04, C12N 5/10, C07K 1/14

(54) **A COST-EFFICTIVE METHOD FOR EXPRESSION AND PURIFICATION OF RECOMBINANT PROTEINS IN PLANTS**
KOSTENGÜNSTIGES VERFAHREN ZUR EXPRESSION UND REINIGUNG REKOMBINANTER PROTEINE IN PFLANZEN
PROCÉDÉ PEU COÛTEUX D'EXPRESSION ET PURIFICATION DE PROTÉINES RECOMBINANTES DANS DES PLANTES

(30) Priority: 21.12.2010 US 201061425703 P
(43) Date of publication of application: 30.10.2013
(73) Proprietor: The Chinese University Of Hong Kong, Hong Kong (CN)
(72) Inventor: SUN, Samuel, Sai Ming, N.T. Hong Kong (CN); TIAN, Li, N.T. Hong Kong (CN)
(74) Representative: Witte, Weller & Partner Patentanwälte mbB
(86) International application number: PCT/US2011/066538
(87) International publication number: WO 2012/088295

(56) References cited:
- US-A1- 2006 263 855
- JURGEN SCHELLER ET AL: "Forcing single-chain variable fragment production in tobacco seeds by fusion to elastin-like polypeptides", PLANT BIOTECHNOLOGY JOURNAL, vol. 4, no. 2, 1 March 2006 (2006-03-01), pages 243-249, XP55111050, ISSN: 1467-7644, DOI: 10.1111/j.1467-7652.2005.00176.x
- FONG BALEY A ET AL: "Expression and purification of ELP-intein-tagged target proteins in high cell density E. coli fermentation", MICROBIAL CELL FACTORIES, BIOMED CENTRAL, LONDON, NL, vol. 9, no. 1, 19 October 2010 (2010-10-19), page 77, XP021077225, ISSN: 1475-2859, DOI: 10.1186/1475-2859-9-77
- FLOSS D M ET AL: "Elastin-like polypeptides revolutionize recombinant protein expression and their biomedical application", TRENDS IN BIOTECHNOLOGY, ELSEVIER PUBLICATIONS, CAMBRIDGE, GB, vol. 28, no. 1, 1 January 2010 (2010-01-01), pages 37-45, XP026813423, ISSN: 0167-7799 [retrieved on 2009-11-10]
- MEYER D E ET AL: "Protein purification by fusion with environmentally responsive elastin-like polypetide: effect of polypeptide length on the purification of thioredoxin", BIOTECHNOLOGY PROGRESS, AMERICAN INSTITUTE OF CHEMICAL ENGINEERS, US, vol. 17, no. 4, 1 January 2001 (2001-01-01), pages 720-728, XP002955911, ISSN: 8756-7938, DOI: 10.1021/BP010049O
- TIAN L ET AL.: 'A Cost-Effective ELP-Intein Coupling System for Recombinant Protein Purification from Plant Production Platform.' PLOS ONE vol. 6, 31 August 2011, pages 1 - 11, XP055108859

## Description

### Technical Field

The present invention provides a cost-effective and efficient method to express and purify recombinant proteins in transgenic plants. The recombinant proteins are expressed as fusions with an ELP-intein tag and include pharmaceuticals, antibody chains and other useful proteins. They may be produced in various plant parts, such as leaves, seeds, roots, tubers, fruits and cell cultures.

### Background art

Using transgenic plants for large-scale production of pharmaceutical proteins has been demonstrated as an attractive system with the advantages of low cost, high yield, easy harvest and reduced health risks in comparison to traditional microbial and mammalian bioreactors, and many valuable recombinant therapeutic proteins have been expressed in transgenic plants as proof-of-concept and feasibility demonstrations (Giddings, G., et al, Nat. Biotechnol.(2000) 18:1151-1155; Twyman, et al, Trends Biotechnol. (2003) 21:570-578; Ko, K., et al., Curr. Top. Microbiol. Immunol. (2009) 332:55-78)). For further development of plant bioreactors, downstream purification of target proteins from plant samples, which is estimated to account for 80% of the production costs in the case of a therapeutic protein (Walter, J. K., et al, In Subramanian, G (ed.) Bioseparation and Bioprocessing, Wiley-VCH, Weinheim, Vol. 1:465-496 (1998)), is the most important factor in large-scale industrial production of recombinant proteins by plant-based bioreactors.

Traditional protein purification methods involve expression of target proteins as fusions to affinity tags, such as the His tag, glutathione S-transferase tag, c-myc tag, strepll epitope, calmodulin-binding peptides and maltose-binding protein (Desai, U. A., et al, Protein Expr. Purif. (2002) 25:195-202; Witte, C. P., et al., Plant Mol. Biol. (2004) 55:135-147; Rubio, V., et al., Plant J. (2005) 41:767-778; Valdez-Ortiz, A., et al., J. Biotechnol (2005) 115:413-423; Streatfield, S. J., Plant Biotechnol J. (2007) 5:2-15). These suffer from the difficulty and high cost of scaling-up of the required affinity chromatography. Several new strategies have emerged to simplify downstream protein purification. Oleosin fusions (Parmenter, D. L., et al., Plant Mol. Biol. (1995) 29:1167-1180; Bhatla, S. C., et al., Biotechnol Adv. (2010) 28:293-300) target the desired protein to an oil body which can be easily separated from water-soluble fractions. Hydrophobin fusions (Lahtinen, T., et al., Protein Expr. Purif. (2008) 59:18-24), alter the hydrophobicity of the fusion protein, and then facilitate the purification by a surfactant-based aqueous two-phase system. Fusion to γ-zein domain (Torrent, M., et al., Methods Mol Biol (2009) 483:193-208) enables the recombinant protein to form dense, ER-localized protein bodies containing a high concentration of the desired protein, which can be readily isolated from cellular material using density-based separation methods. However, because fusion tags may affect the bioactivity of recombinant proteins, they need to be removed and this generally depends on proteolytic cleavage by a protease. This additional cleavage step results in higher cost due to the cost of protease and of an extra step in its removal, in addition to the potential occurrence of non-specific protein cleavage. The development of a simple and cost-effective downstream recombinant protein purification system is thus highly desirable.

The elastin-like polypeptide (ELP) -intein system is a simple and efficient method for purification of proteins from *E. coli* (Kang, H. J., et al., J. Microbiol Biotechnol (2007) 17:1751-1757; Lim, D. W., et al., Biomacromolecules (2007) 8:1417-1424; Floss, D. M., et al., Trends Biotechnol (2010) 28:37-45; Hu, F., et al., Appl Biochem Biotechnol (2010) 160:2377-2387. ELP protein consists of repeating pentapeptides of V-P-G-X-G (X can be any amino acid except proline) and has an attractive property of temperature-sensitive phase transition: when temperature is increased to its transition temperature (Tt), soluble ELP will aggregate into insoluble phase which can be precipitated easily into a pellet by centrifugation; but when cold buffer is added, aggregated ELP can be resolubilized and returned to soluble phase. The Tt can be regulated by salt concentration, temperature and the length and component of the repeating pentapeptides. ELP has the advantages of low-cost and easy scale-up.

Intein is a kind of protein splicing element which catalyzes self-cleavage and, with substitution of some of its amino acids, intein can be regulated to cleave at its N- and/or C-terminus in response to pH shifts or thiol reagents. Inteins exist in a large number of proteins and catalyze self-cleavage of a "pro" form to the mature protein. Many inteins have been identified and a catalog of such sequences may be found at the web site "neb.com" under the designation "neb/inteins". The self-cleavage property of intein can thus be applied to replace proteolytic cleavage. Description of many intein proteins have also been published: (Perler, F. B., Nucleic Acids Res. (1999) 27:346-347 and used as fusion partners of ELP or other affinity tags (Fong, B. A., et al., Protein Expr Purif (2009) 66:198-202; Gillies, A. R., et al., Methods Mol. Biol. (2009) 498:173-183; Srinivasa Babu, K., et al., Biotechnol Lett (2009) 31:659-664; Hong, I., et al., J. Microbiol Biotechnol (2010) 20:350-355; Ma, C., et al., Protein Pept Lett. (2010) 17:1245-1250.

ELP-intein (Ei) fusion strategy is thus an attractive method for protein purification. After several cycles of ELP phase transition, the fusion protein will be separated from other proteins through temperature shift and centrifugation. Cleavage of intein can then be triggered by a pH shift or chemical addition to cleave the desired protein from the Ei tag, followed by another phase transition of ELP to separate the desired protein supernatant and the Ei tag as a pellet. No additional protease or special equipment are needed in the whole procedure, thus reducing the purification cost and largely simplifying the operation. A U.S. patent publication No. 20060263855 (Wood) discloses the purification of recombinant protein from *E. coli* cells by an ELP-intein fusion system.

In the last twenty years, application of ELP or intein in the alternative in transgenic plants has been studied (Morassutti, C., et al., FEBS Lett (2002) 519:141-146; Scheller, J., et al., Plant Biotechnol J. (2006) 4:243-249; Lao, U. L., et al., Biotechnol. Bioeng. (2007) 98:349-355; Patel, J., et al., Transgenic Res. (2007) 16:239-249; Conley, A. J., et al., Biotechnol Bioeng (2009a) 103:562-573; Conley, A. J., et al., BMC Biol (2009b) 7:48; Floss, *et al*., *supra* (2010)).

ELP fusion was reported to enhance the expression of recombinant proteins in transgenic seeds and leaves (Scheller, J., et al., Transgenic Res (2006) 13:51-57; Patel, *et al*., *supra* (2007)), and the length of ELP was indicated to have different effects on recombinant protein accumulation and protein recovery during the inverse transition cycling (Conley, *et al*., *supra* (2009a)). In addition, Conley, *et al*., *supra* (2009b) had evaluated the effects of ELP fusion on the accumulation of GFP targeted to the cytoplasm, chloroplast, apoplast and endoplasmic reticulum (ER).

Morassutti, C., *et al*., *supra* (2002) provided the first evidence of production of a recombinant protein in transgenic plants by exploiting the intein-mediated self-cleavage mechanism.

However, all of these reports used either ELP or intein in the alternative as fusion tags. Applicants are not aware of any report on the use of ELP and intein in combination in transgenic plants.

It has been reported that ELP fusion proteins form protein bodies in tobacco leaves (Conley, A.J., *et al*., *supra* (2009b)). We have also found recombinant protein fused to the ELP-intein tag formed ER-derived protein bodies in rice seeds (Tian, L., *et al.* unpublished). In view of the hydrophobic nature of ELP, ELP fusion proteins are likely to form protein bodies, leading to difficulty in extraction of soluble ELP fusion protein for further purification. Routine optimization of extraction and purification procedures, such as extraction buffer, extraction methods and purification operation may be needed to purify target proteins from plant samples by the ELP-intein system.

Although many previous reports on ELP fusion expression of recombinant proteins in plants had demonstrated that ELP fusion proteins could be purified, most of these experiments were performed in tobacco leaves by transient gene expression which might lead to high accumulation of heterogeneous proteins temporarily. However, when we initially tried to express and purify a recombinant protein, human granulocyte colony stimulating factor (hG-CSF), fused to an ELP-intein tag from tobacco leaves stably transformed, the expression was very low and purification encountered great difficulty in recovering significant amounts of the target protein (Tian, L., *et al*., unpublished). It thus seems that ELP fusion expression is quite different from ELP-intein fusion, and the situations of ELP-intein fusion expression and purification in stable plant transformants appear to be more complicated. Again, routine optimization of conditions should be employed.

### Disclosure of the Invention

The invention provides a cost-effective, efficient and scalable method to express and purify recombinant proteins from plants. The invention is also directed to the ELP-intein fused proteins, recombinant materials and methods for their production and plant transformants.

The invention takes advantage of an ELP-intein fusion expression and purification system to purify recombinant proteins from plant samples. Such recombinant proteins include pharmaceuticals, antibody chains, industrial enzymes and any other useful proteins. These may be produced in any plants and various plant parts, such as calli, leaves, seeds, roots, tubers, fruits and cell cultures. The method is simply operated and can be easily scaled up for industrial production through temperature-sensitive inverse phase transition of ELP, without the need of affinity chromatography and special equipment. Compared to traditional purification methods of fusion expression strategy applied in plants, the invention method utilizes self-cleaving intein protein to release the desired recombinant protein from the fusion tag, thus avoiding the use of protease for fusion tag cleavage.

In one aspect the present invention is directed to a method to obtain a desired protein from plants, plant parts, or plant cells according to claim 1.

In other aspects, the invention is directed to recombinant expression systems operable in plants for said fusion protein and to plants, plant parts or plant cells express said fusion protein as well as methods to produce said fusion proteins in plants, plant parts or plant cells.

### Brief Description of Drawings

Figures 1(a)-1(c) show information on a transformation plasmid exemplified below.
Figures 2(a)-2(b) show SDS-PAGE and Western Blot analysis of ELP-intein-PAL fusion protein accumulated in mature transgenic rice seeds.
Figure 3 shows a purification scheme of ELP-intein-PAL fusion protein from rice seeds.
Figure 4 shows successful PAL purification from transgenic rice seeds by the ELP-intein system.
Figures 5(a)-5(e) show a graphical representation of the effects of purified PAL according to the method of the invention on various cell lines.
Figure 6 shows a diagram of the expression system used to generate a fusion protein comprising hG-CSF in tobacco.
Figures 7(a)-7(c) show SDS-PAGE results of expression of human G-CSF as a fusion protein in tobacco leaves.
Figure 8 shows a diagram of the steps in purification of hG-CSF from tobacco seeds when produced as a fusion protein.
Figure 9 shows the results according to SDS-PAGE of the purification of hG-CSF from fusion protein expression in tobacco leaves.

### Modes of Carrying Out the Invention

The invention couples target gene expression with a downstream purification method in plant-based production of recombinant proteins. The plant samples may be from any kind of plants, monocot or dicot, and various plant parts, such as calli, leaves, seeds, roots, tubers, or cell cultures. Based on the nature of samples, the strategy for the expression and purification may vary. For example, fresh samples, such as leaves and calli, may be preferable for extraction, and further purification in light of their simple lysis, while dry samples, such as seeds, with advantages of low protein degradation, high protein content and convenience in transport, are good starting samples for extraction and purification.

The recombinant proteins produced and purified by the invention methods may be of any peptide sizes, from any sources of mammals, bacteria or plants, and with any function to perform on humans, animals or plants.

The ELP-intein system is employed to express a fusion of the desired protein with an ELP-intein tag, wherein intein is located between ELP and the recombinant protein. The property of temperature-sensitive phase transition of ELP is used to separate fusion protein from other cell components, and the self-cleavage function of intein releases the recombinant protein from the ELP-intein tag. After cleavage, the ELP-intein tag can be converted by the ELP phase transition into an insoluble aggregate, which can be easily separated and removed from the soluble recombinant protein.

The fusion proteins are encoded by an continuous open reading frame of a sequence including ELP-encoding, intein-encoding and recombinant protein-encoding gene sequences, wherein intein gene is inserted between ELP and recombinant protein gene sequences. An appropriate linker sequence encoding a short amino acid sequence may, of desired, be inserted between ELP and intein or intein and recombinant protein or even among ELP units, to allow necessary folding of ELP, intein and recombinant protein.

One ELP protein domain may contain one or more ELP units, *i.e*., V-P-G-X-G peptides, where X is any amino acid except proline. The repetitive number of ELP units may influence the phase transition temperature of ELP protein, therefore the length of ELP domain *(i.e.,* the number of ELP units) may be adjusted for practical demand. The number of ELP units may vary from one to several hundred; thus, ELP domains with 5, 10, 20, 30, 50, 100 or more units may be employed. The intermediate numbers are also included. The fusion protein contains at least one ELP domain, but may contain more than one. For example, two ELP domains may be separately located at the N- and C-termini of the recombinant protein with the intein proteins between the termini and the ELP domains.

Intein is generally located between ELP and recombinant protein, and the cleavage site, N- or C-terminus of intein, may be modulated by substitution of some amino acids in intein protein. The cleavage reaction can be effected by changes in environmental conditions such as pH, addition of thiol reagents and an increase in temperature. The cleavage of intein at its C-terminus to release recombinant protein from ELP-intein may be effected by pH alteration from 8.5 to 6.0-6.5. In another variant, intein is cleaved at its N-terminus by addition of a thiol reagent.

The first or last amino acid of the target protein, connected to the cleavage site of intein, may have a role in cleavage reaction and determine the efficiency of cleavage. Therefore, to make the cleavage work, consideration should be given to changing or maintaining the first/last amino acid of the target protein or adding another amino acid. In one variant, an amino acid Met is added to the N-terminus of recombinant protein, *i.e.,* the *Pandanus* lectin (PAL). Such modifications are not limited to this particular protein. They may be beneficial regardless of the nature of the desired protein. If the tag is at the C-terminus, modification of the C-terminal amino acid may also be desirable.

The level of recombinant protein accumulation in plants always plays an important role in its subsequent downstream purification. Generally, high accumulation is preferable for purification. Therefore, optimization of any components of the plasmid construction may be made to increase protein expression and facilitate the accumulation of the target protein in a specific organ, tissue or intracellular location, through using strong promoters, optimizing codon usage and/or targeting the protein to a specific cell compartment by targeting signals. In one embodiment for optimizing expression, a ubiquitin fusion strategy is used.

Nucleic acid sequences encoding fusion proteins for construction of fusion expression plasmids, comprise an open reading frame encoding the ELP-intein fusion protein, wherein the sequence encoding ELP-intein fusion tag may be located at the N- or C-terminus of the recombinant protein. Any linker sequences may be located between ELP and intein and/or between intein and the recombinant protein and/or the ELP units themselves, but the sequence of the linker is selected so as not to affect the folding of the fusion protein or any of its components. The sequences encoding ELP and intein protein domains may be optimized to improve the expression and purification of recombinant protein, by controlling the number of ELP units and making amino acid substitutions in intein protein. Expression may be optimized at transcription, translation and post-translation levels and targeting location effected by choice of promoter, any signal peptide or targeting peptide applied, the 5' UTR component, codon usage and other fusion compositions.

Various transformation methods leading to transient or stable expression of ELP-intein fusion proteins, such as *Agrobacterium*-mediated transformation and biolistic particle delivery systems may be used.

Extraction and purification of desired proteins from plant samples may be routinely optimized by methods that comprise:
(a) optimization of extraction technology, including, but not limited to extraction buffer, instrument applied, extraction temperature, sample lysis and pre-purification of any tissues based on the location of the fusion protein;
(b) pre-treatment of crude extraction samples before purification by ELP phase transition, including removal of any insoluble and other nonspecific components;
(c) adjustment of the conditions to trigger ELP phase transition, including temperature, pH, salt component, equipment applied, additional ELP protein or any other component additions;
(d) choice of method to separate aggregated ELP fusion proteins from other contaminant components, such as microfiltration, centrifugation or any other methods leading to separation;
(e) modifications of methods to resolubilize the aggregated ELP fusion protein, including temperature, buffer component, pH and any equipment applied;
(f) modifications of the operation of ELP phase transition to obtain pure ELP-intein fusion protein, such as the cycling of ELP phase transition, combination of different methods related to ELP-based purification in (c), (d) and (e) mentioned above;
(g) adjustment of the conditions to trigger intein-induced cleavage, such as buffer component, pH, temperature and chemical addition; and
(h) optimization to separate final cleaved recombinant proteins from the cleaved ELP-intein tag after intein-induced cleavage, such as related ELP phase transition and any further purification.

At times, self-cleavage of intein may occur *in vivo,* especially for the inherent cleavage function in response to pH, which may vary due to intracellular location or even the plant growth environment. In the example below, there was partial cleavage of ELP-intein-PAL fusion protein *in vivo.* However, the *in vivo* cleavage did not affect the purification of the uncleaved fusion protein.

The method can be performed to purify recombinant proteins from both transient and stable expression. Transformation methods can also be adjusted and conducted according to practical situation, such as experimental conditions and host plants.

After expression, protein extraction is one of the key factors in the subsequent purification steps. Routine optimization of extraction conditions may be performed, including buffer components, pH, temperature, equipment applied to lyse cells and necessary gradient separation. Extraction methods can also be adjusted based on the intracellular location of the target protein as is known in the art. In the exemplified aspect of the present invention, seed powder was used for extraction and urea was added to the extraction buffer to extract the fusion protein aggregated with endogenous prolamins in the protein bodies of rice seeds.

Before purification by the ELP-intein system, pre-treatment may need to be conducted to remove any insoluble compositions by filtration, centrifugation or other possible procedures. If some components exist in the extraction buffer affecting the solubility of fusion protein or the following ELP phase transition or intein cleavage, pre-clearance may be effected through desalting, dialysis, filtration and further centrifugation. In the exemplified aspect of the present invention, urea in the extraction buffer was removed by desalting or dialysis.

After sample extraction, purification via the ELP-intein system is initiated. As mentioned in the above Background, the phase transition temperature of ELP is influenced by many factors, including the length and component of the ELP protein, the concentration of ELP fusion protein, the size and nature of intein and the recombinant protein, the temperature and the salt concentration and composition. To effect aggregation of ELP, conditions can be adjusted and optimized, preferably, with mild temperature and buffer components.

Following the aggregation step, the ELP-intein fusion protein aggregate is resolubilized. Generally, low temperature and cold buffer help the aggregated fusion protein return to its soluble phase. Optimization may be performed by adjusting the components of resolubilization buffer, prolonged ice bathing or low temperature incubation, or even by the help of equipment, such as microfiltration and shaking. After resolubilization of aggregated fusion protein, further centrifugation may be needed to remove any insoluble components. Microfiltration is used to replace the first cycle centrifugation so as to facilitate the recovery of fusion proteins while normal centrifugation is may be applied in the second and third cycle. In another variant, agitation of 4°C, for example, overnight enhances ELP resolubilization.

In the integrated procedure to separate ELP fusion protein from other contaminant proteins, the steps from aggregation of soluble ELP fusion protein to recovery of the aggregated fusion protein into soluble state are defined as one separation cycle. To obtain pure fusion protein, the cycle may be repeated several times, and the separation operation in the cycles may be adjusted with different methods, such as through microfiltration and/or centrifugation. However, while extensive and longer separation procedures may give higher purity, the yield of fusion proteins may be reduced.

Once ELP-intein fusion protein with the desired purity is obtained, the cleavage reaction of intein can be triggered by changing reaction pH and/or temperature and/or by adding other chemicals such as adjusting salt concentration. Additional complementary operations may be applied to accelerate the cleavage, such as by prolonging the reaction time and freeze-thawing. The cleavage ratio can be estimated by SDS-PAGE or other methods.

Preferably complete cleavage is achieved before the final separation of the cleaved ELP-intein tag from the released recombinant protein. The ELP-intein tag and recombinant protein are separated in a similar manner by aggregating the ELP-intein portion and removing it from the soluble protein in the supernatant. Final product of the desired recombinant protein may be further dialysed or desalted to remove any undesirable small components in the buffer applied.

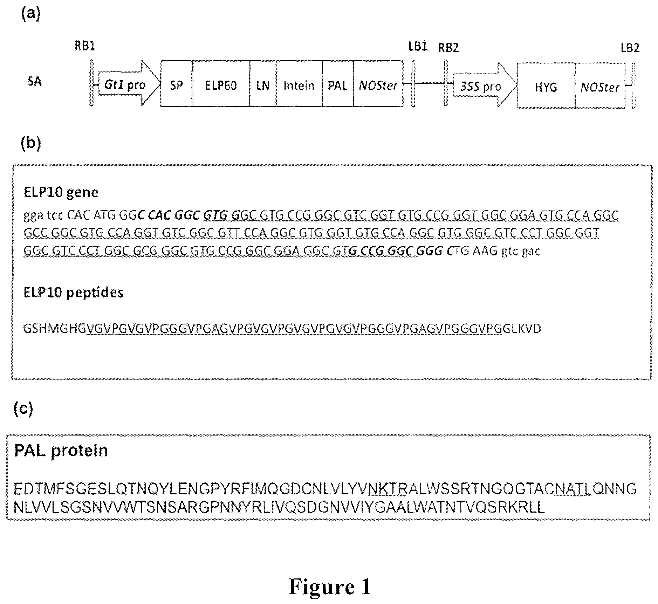

In one example below, transgenic rice seed was used as a production system for *Pandanus* lectin (PAL), which was expressed as a fusion to an ELP-intein tag. PAL protein is a monocot mannose-binding lectin from *Pandanus amaryllifolius.* The PAL protein, with a molecular weight (Mw) about 12.5 kD (protein sequence shown in Figure 1(c)), exhibits inhibitory activity on the proliferation of several human cancer cell lines (Tian, L., *et al*., unpublished).

The applicability of the expression of the fusion protein and the purification system of the invention is also demonstrated in tobacco as set forth below.

The following examples are offered to illustrate but not to limit the invention.

### Example 1

### Recombinant Anti-Tumor Protein Purification from Transgenic Rice Seeds

In this example, transgenic rice seeds were used as a production system and a *Pandanus* lectin (PAL) with a molecular weight (Mw) about 12.5 kD (protein sequence shown in Figure 1c) as a target protein. PAL exhibits inhibitory activity on the proliferation of several human cancer cell lines (HepG2, A549, DLD-1, U87 and Capan-2). PAL was expressed in fusion to ELP-intein tag, wherein ELP contained 60-repeat "VPGXG" peptides, and the intein protein is cleaved at its C-terminus in response to low pH. The ELP-intein tag was inserted at the N-terminus of PAL, and after purification of the ELP-intein-PAL fusion protein from rice seeds, intein cleavage could be triggered by decreasing the buffer pH to separate the target PAL protein from Ei tag.

The expression cassette shown in Figure 1(a) for the ELP-intein-PAL fusion protein is driven by rice glutelin *Glu*A (Gt1) promoter and signal peptide. This sequence was cloned into the multiple cloning sites of the T-DNA binary vector pSB130 for rice transformation. The construct was named SA.

The ELP60 domain-encoding sequence was generated from six repeats of the ELP10 gene shown in Figure 1(b) by similar methods as described by Scheller, J., et al. (Transgenic Res. (2006) 13:51-57). The Ssp DnaB intein (referenced as intein1) and linker gene were obtained from the pTWIN2 vector (5902-5940 bp for linker gene and 5941-6402 bp for intein gene) purchased from NEB found on the World Wide Web at neb.com/nebecomm/products/productN6952.asp). Both ELP10 and intein genes were optimized for preferred codon usage of rice and synthesized by GenScript Corporation.

*Agrobacterium*-mediated transformation was carried out using rice cultivar 9983 with the vector construct mentioned above. Rice seed calli induction, *Agrobactrium*-midated tranformation, selection and regeneration of rice plants were performed following the protocol provided by CAMBIA on the World Wide Web at cambia.org/daisy/cambia/4214.html. Regenerated transgenic rice plantlets were transferred to soil and grown in facilities for transgenic plants at the Chinese University of Hong Kong. After primary screening by PCR on the regenerated rice plants, integeration of transgenes into the genome of rice plants was further confirmed by Southern blot. Several transgenic plants containing one to two copies of transgenes were obtained. Mature positive transgenic rice seeds were collected as T1 seeds. T2 seeds generated from positive T1 plants were used for protein analysis by SDS-PAGE and western blot using specific antibody.

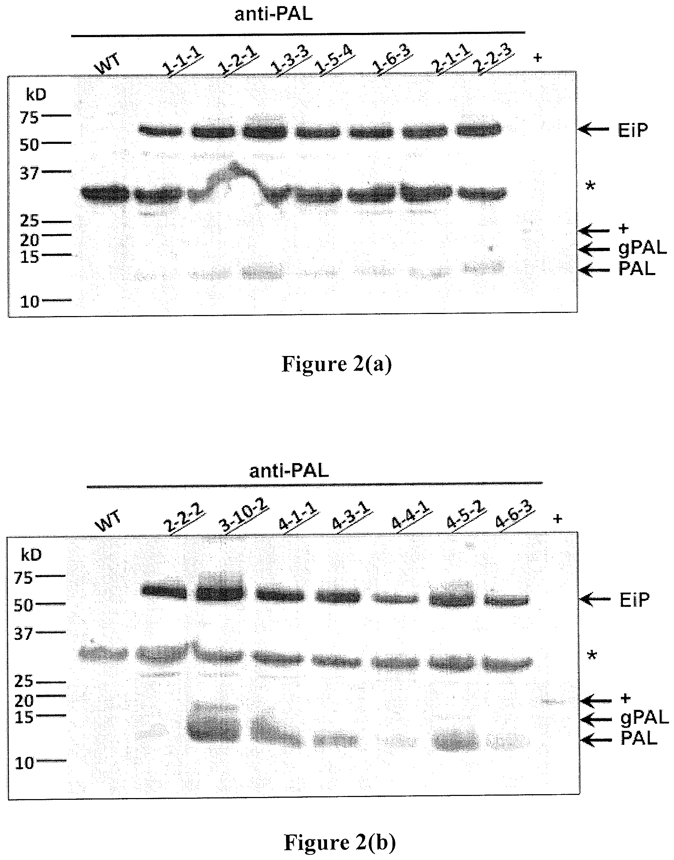

PAL protein expression in transgenic rice seeds was detected by SDS-PAGE and western blot analysis using anti-PAL antibody as shown in Figure 2 and described below.

Mature rice seeds were ground into powder by a blender. Total protein extraction buffer (0.1 M Tris-HCl, pH 8.5, 50 mM NaCl, 5% SDS, 4 M urea, 5% β-mercaptoethanol) was added to the seed powder at 20 µl/mg, and incubated at 35-37°C with intense shaking for 2-4 hours. The whole homogenate was centrifuged at 20,000 × g for 10 minutes at room temperature twice. Supernatant was collected as total extracted protein (TEP) from seeds for expression analysis. For Western blot, TEP was separated by 15% SDS-PAGE using loading buffer (50 mM Tris-HCl, pH 8.5, 2% SDS; 10% Glycerol; 0.02% Bromophenol Blue) and transferred to PVDF membrane. Western blot was carried out using anti-PAL primary antibody from rabbit and anti-Rabbit IgG-Peroxidase antibody (Sigma). Recombinant PAL produced by *E. coli* was used as positive control.

In SA transformed rice seeds, the fusion protein with expected Mw (56 kD) could be observed in SDS-PAGE analysis. Some fusion protein was self-cleaved *in vivo* in the SA seeds, resulting in free PAL and glycosylated PAL was detected. Western blot analysis on the total seed protein from independent rice lines is shown in Figures 2(a) and 2(b). When focusing only on the *in vivo* uncleaved EiP fusion protein determined by Western blot, the levels of EiP protein accumulation in SA transgenic rice seeds on average amounted to 1.40 mg/g dry seeds.

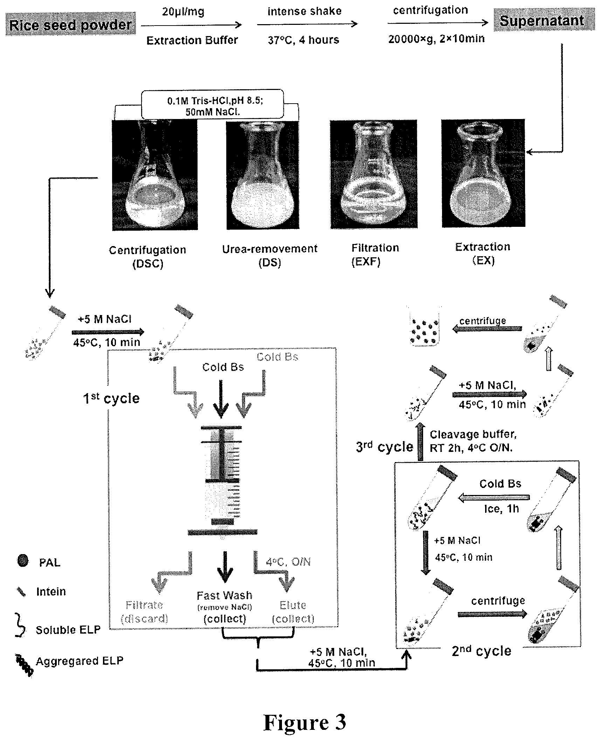

The procedure from total protein extraction to final purification is shown schematically in Figure 3. Generally, two or three cycles of phase transition of ELP were needed to obtain pure EiP protein, and about 2-3 days were required due to overnight incubation in two of the steps.

In more detail, total protein was extracted by Bt buffer (0.1 M Tris-Cl, pH 8.5; 50 mM NaCl; 6 M Urea; 0.5% Tween™-20). The extraction sample (EX) was filtered (EXF) to remove any debris, desalted (DS) by PD-10 column (GE Healthcare) or dialysis with Bp Buffer (0.1 M Tris-HCl, pH 8.5; 50 mM NaCl) to remove urea, and then centrifuged (DSC) at 20,000 × g for 10 min at 4°C to remove any insoluble debris. The supernatant was collected for the purification.

To trigger the temperature-sensitive inverse phase transition of ELP, 5 M NaCl was added to the samples at a ratio of 1:1 (v/v) and the mixture was incubated at 45°C for 10 min. The 1^{st} purification cycle was performed as described by Ge, et al. (Biotechnol Bioeng (2006) 95:424-432) with some modifications. The NaCl-treated sample was transferred to a syringe equipped with a Millipore™ filter (PES membrane, 0.22 µm) and the filtrate was discarded while the aggregated ELP-intein-PAL fusion protein remained in the filter. One ml cold Bs buffer (0.1 M Tris-HCl, pH 8.5; 50 mM NaCl; 0.1% Tween™-20) was passed through quickly to remove additional NaCl from the filter and the wash was collected as some fusion protein might return to soluble state and passed the filter. Another 1-3 ml (or 1/10 volume of original TEP sample) cold Bs buffer was added into the syringe without a plunger, and the whole system was kept at 4°C overnight with a tube below the filter to collect the eluate (soluble EiP) by gravity. Any remaining solution was pushed by a plunger to collect the rest of the soluble EiP. The eluate and the wash were combined as 1CS sample.

The 2nd cycle was carried out by the inverse phase transition procedure as reported by Wu, et al. (Nature Protoc. (2006) 1:2257-2262). NaCl (5 M) was added at a ratio of 1:1 v/v, and the mixture was incubated at 45°C for 10 min, followed by immediate centrifugation. The pellet was resuspended in cold Bs buffer with 1/5 original volume and kept on ice for 1 hour with gentle agitation. After centrifugation at 4°C, the supernatant was collected as 2CS sample. The 3rd cycle was performed as the 2nd cycle except that 50 mM PBS buffer (pH 7.2) was used to resolubilize the pellet. The supernatant was collected as 3CS sample.

To trigger the cleavage reaction by intein, 1/10 volume of 1 M Tris-HCl (pH 4.5) was added into the 3CS sample to a final pH value at 6.0-6.5. The sample was allowed to cleave at room temperature for 2 hours and then 4°C overnight (for complete cleavage). Another phase transition was performed by addition of NaCl followed by centrifugation. The supernatant was collected as final purified target protein (FS) and the pellet was re-suspended as final ELP-intein tag (FP).

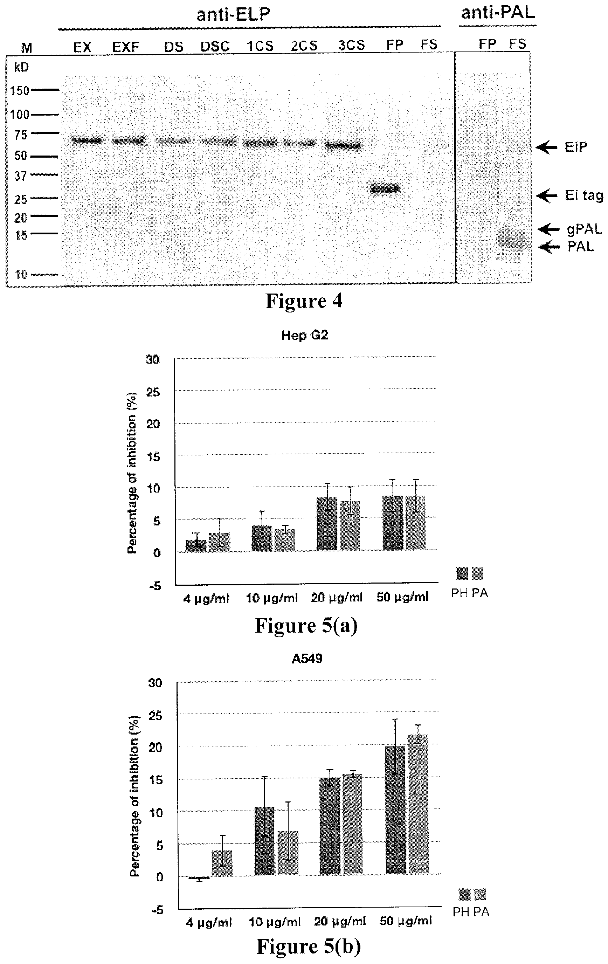

Purified EiP fusion protein could be observed in SDS-PAGE after 2 or 3 cycles. In the cleavage step, pH shift achieved by adding Tris buffer at low pH initiated the intein cleavage reaction, but longer incubation, such as 24 to 36 hours at 4°C is used for complete cleavage. Freeze-thaw treatment can be used to accelerate the cleavage. The cleavage and the subsequent purification steps were efficient as no ELP-intein tag remained in the final supernatant of PAL (Figure 4, lane FS). By this highly optimized method, the yield of pure PAL protein was about 30-80 µg/g dry seeds.

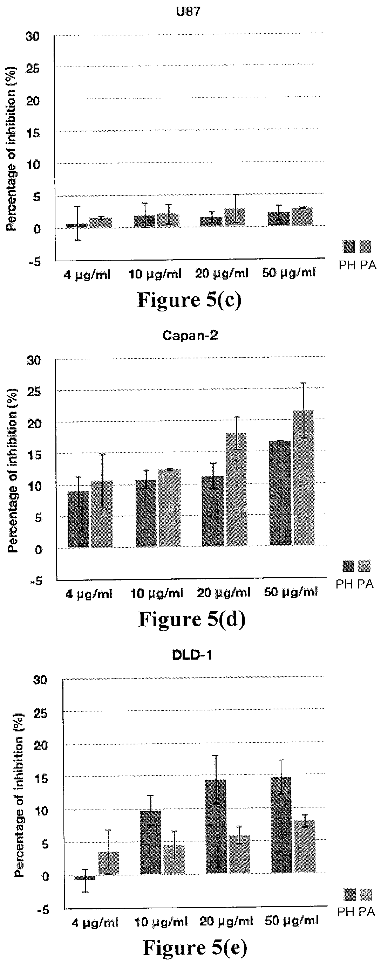

PAL belongs to monocot mannose-binding lectins and behaves inhibition activity on the proliferation of human cancer cells (Figure 5, Sample PH). We tested the bioactivity of PAL protein purified by the ELP-intein system from rice seeds using the proliferation inhibitory activity analysis on several different human cancer-cell lines, HepG2 (liver), A549 (lung), DLD-1 (colon), U87 (glioblastoma) and Capan-2 (pancreas). Results showed that the PAL purified by the ELP-intein system exhibited similar dose-dependent inhibition activity to PAL protein purified from *E. coli* (as shown in Figure 5), indicating that the ELP-intein fusion expression and purification system did not diminish the bioactivity of the recombinant protein.

### Example 2

### Recombinant Human G-CSF Purification from Transgenic Tobacco

In this embodiment, transgenic tobacco leaves were used as a production system while human granulocyte colony-stimulating factor (hG-CSF), an important human cytokine which has been widely used in oncology and infection protection, was used as a target protein.

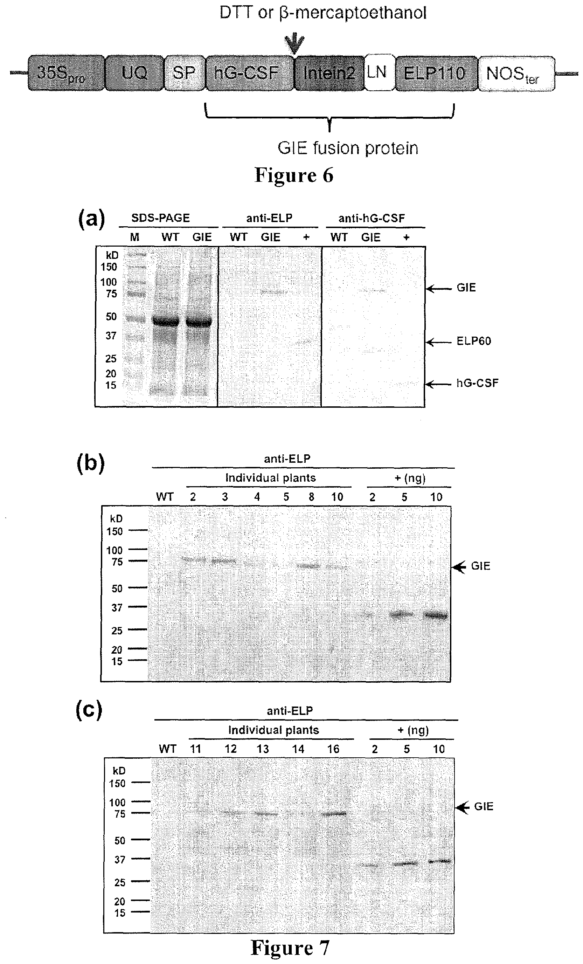

The *CaMV* 35S promoter in binary vector pBI121 was used to construct hG-CSF fusion expression chimeras and the phaseolin signal peptide was introduced to direct the expressed fusion protein into the plant cell secretory pathway. The expression cassette is shown in Figure 6. Ubiquitin was introduced to improve the expression of the target protein and would be processed accurately from the fusion protein by endogenous ubiquitin-specific proteases (Ubps) (Tian, L. and Sun, S.S.M., *BMC Biotechnol* (2011) 11:91). Figure 6 shows the sequences encoding the hG-CSF-intein2-ELP110 fusion protein denoted by the bracket, and the arrow denotes the cleavage site of intein2 triggered by addition of DTT or β-mercaptoethanol.

The hG-CSF was expressed in fusion with an intein-ELP (IE) tag at the C-terminus, shown as hG-CSF-intein-ELP or "GIE" (Figure 6), wherein ELP contained 110 repeating "VPGXG" peptides while intein protein (referenced as intein2) is cleaved at its N-terminus in response to the addition of thiol reagents, such as dithiothreitol (DTT) or β-mercaptoethanol. After purification of the GIE fusion protein from tobacco leaves, intein cleavage was triggered by adding β-mercaptoethanol to release the target hG-CSF protein from the IE tag.

The ELP110 gene was generated from 11 repeats of the ELP10 gene (Figure 1(b)) by similar methods as described above. The Mth RIR1 intein and linker genes were obtained from the pTWIN2 vector (6445-6846 bp for intein gene and 6847-6888 bp for linker gene) purchased from NEB on the World Wide Web at neb.com/nebecomm/products/productN6952.asp.

The chimeric genes in pBI121 expression vectors were transformed into *Agrobacterium tumefaciens* LBA4404 by electroporation and *Agrobacterium*-mediated transformation was carried out using young tobacco leaves as described previously (Tian, L. and Sun, S.S.M, *BMC Biotechnol* (2011) 11:91). After plant regeneration, individual plants were PCR screened using hG-CSF specific primers and 11 of 15 transfected plants showed positive results.

To detect expression of GIE proteins in transfected plants, total soluble protein was extracted from fresh young leaves of 21-day-old transgenic plants with extraction buffer [0.1 M phosphate buffered saline (PBS), pH 7.2; 1 mM EDTA; 0.1% Tween™-20; 100 mM ascorbic acid; 2% polyvinylpyrrolidone; complete protease inhibitor (cocktail tablets, Roche)]. Fresh leaves (1 g) were ground into powder in a mortar with liquid nitrogen and 1 ml extraction buffer was added in. Whole homogenate was transferred into 2 ml Eppendorf tube, incubated on ice for 15 min and centrifuged at 20,000 × g, 4°C for 10 minutes. Supernatant was collected and centrifuged for another 10 minutes. Final supernatant was collected as total soluble protein from leaves.

Total soluble protein extracted from leaves was diluted with 4 × loading buffer (0.2 M Tris-HCl, pH 6.8; 0.8 g SDS; 40% glycerol; 5% β-mercaptoethanol; 50 mM EDTA; 8 mg Bromophenol Blue), boiled for 5 minutes and separated on 15% SDS-PAGE with 10-50 µg protein/lane followed by Coomassie® Brilliant Blue Staining for protein visualization. For immunoblot, the total protein separated by SDS-PAGE was directly transferred to PVDF membrane without staining. Western blot was carried out using rabbit polyclonal anti-hG-CSF antibody (PeproTech) and anti-ELP antibody as primary antibodies and anti-rabbit IgG-peroxidase antibody (Sigma) as secondary antibody and developed using the ECL detection system (Amersham Co., Bucks, UK). Recombinant hG-CSF purchased from PeproTech and ELP60 protein produced by *E. coli* were used as positive controls.

As shown in Figure 7(a), GIE fusion protein with expected molecular weight (80 kD) was synthesized without self-cleavage *in vivo* in transgenic tobacco leaves, although no distinct difference in protein banding patterns was observed between wild-type (WT) and transgenic plants through SDS-PAGE. Western blot analysis on the total soluble protein from individual plants is shown in Figures 7(b) and 7(c). The levels of GIE protein accumulation in transgenic tobacco leaves on average amounted to 126 µg/g fresh weight (FW).

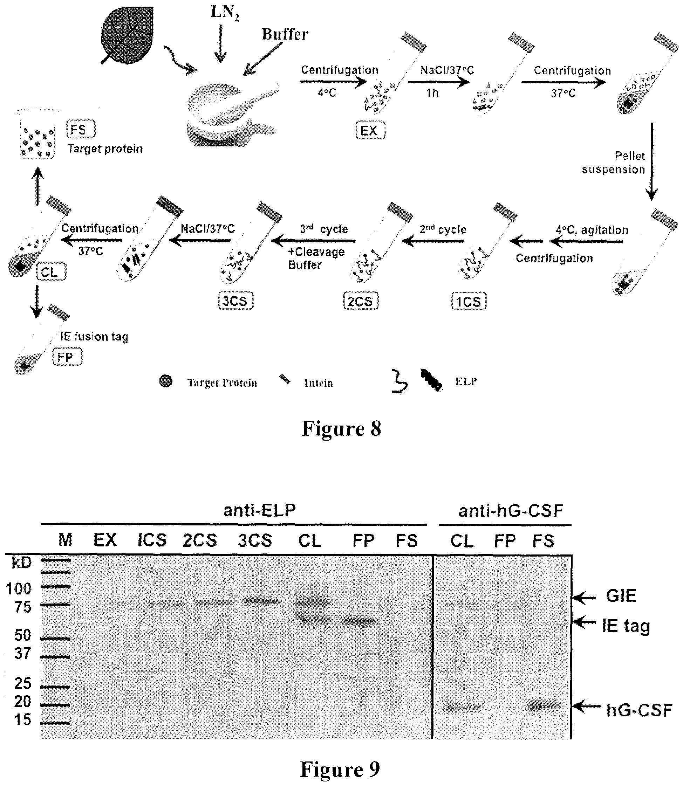

The procedure to obtain purified target protein starting from total soluble protein extraction to final purification is shown schematically in Figure 8. Generally, three or more cycles of phase transition of ELP are needed to obtain pure GIE protein, lasting 2 or more days due to overnight incubation in two of the steps.

In this example, we used ELP consisting of 110 VPGXG repeats to improve the efficiency of ELP phase transition. Because of the low accumulation level of GIE fusion protein in tobacco leaves, to trigger GIE aggregation during purification, addition of the same volume of 3 M NaCl and 37°C incubation for 1-3 hours were used. After aggregation of GIE protein followed by centrifugation, re-solubilization of the target fusion protein in the 1st cycle was performed by adding suspension buffer (50 mM phosphate buffered saline (PBS), pH 7.2; 0.1% Tween™-20; complete protease inhibitor) and agitating under 4°C for overnight. The suspension was centrifuged at 2000 × g, 4°C for 20 minutes and the supernatant was collected as sample 1CS.

Similar purification methods were used in the 2nd and 3rd cycles, except that pre-cleavage buffer (50 mM Tris-HCl, pH 8.5; 50 mM NaCl; 1 mM EDTA) was used to resolubilize the aggregated GIE fusion protein in the 3rd cycle. Along with the purification procedure, the pigments in the extraction were also removed gradually.

To trigger the cleavage of intein, β-mercaptoethanol was added into sample after 3 cycles to a final concentration of 10 mM and the sample was kept at room temperature for 30 minutes and 4°C for 2 hours to complete the cleavage. To monitor the cleavage reaction, partial sample was collected (sample CL) after incubation at room temperature for 10 minutes. After cleavage, another phase transition cycle of ELP was performed as described above and the final cleaved hG-CSF in supernatant (sample FS) and intein-ELP tag in pellet (sample FP) were collected. See Figure 9.

Purified GIE fusion protein could be clearly observed in SDS-PAGE after 3 cycles and the intein cleavage could be triggered to release the hG-CSF from intein-ELP tag. As shown in Figure 9, the cleavage and subsequent purification steps were efficient as no IE tag remained in the final supernatant of hG-CSF (lane FS). Recovery of the final hG-CSF was 10-20 ng per gram fresh leaves and the expression level was 126 µg/g fresh leaves of the target protein.

Human G-CSF is an important pharmaceutical protein, which can be used to reinforce the immune system in patients with human immunodeficiency virus (HIV), pneumonia, diabetic foot infections, leukemia and fibrile neutropenia and to treat cancer patients undergoing chemotherapy to alleviate the depression of white blood cell levels produced by cytotoxic therapeutic agents.

The final supernatant of hG-CSF (sample FS as described above) was desalted, freeze-dried and resuspended in detection buffer (50 mM PBS, pH 7.2) for bioactivity testing which was performed by measuring capability to promote the proliferation of hG-CSF-dependent NFS-60 cell line. This cell line grows only under the presence of hG-CSF or other known growth factors. After 72 hours incubation, the cells treated with detection buffer showed similar baseline proliferation level with the untreated sample. When treated with 1 ng commercial hG-CSF, the proliferation of NFS-60 cells was promoted by 260% over the untreated sample CT while the sample containing detection buffer supplemented with 1 ng purified hG-CSF from tobacco leaves showed similar captivity to promote the proliferation of NFS-60 cells, indicating that the purified hG-CSF was bioactive as the commercial hG-CSF. The purified GIE protein was also tested and showed biological activity but with corresponding decrease in cell proliferation activity, suggesting that the intein-ELP tag did not destroy the bioactivity of the target hG-CSF. These results confirm that the ELP-intein fusion expression and purification system does not diminish the bioactivity of the recombinant protein.

## Claims

1. A method to obtain a desired protein from plants, plant parts or plant cells, which method comprises
(a) preparing, at optimal pH and temperature, an extract of proteins from said plants, plant parts or plant cells, that stably produce said desired protein as a fusion protein with intein and ELP from an optimized recombinant expression system that produces said fusion protein, wherein said fusion protein comprises at either the N-terminus or C-terminus or both of a desired protein, an ELP-intein tag which comprises an elastin-like polypeptide (ELP) domain and an intein splicing element (intein) wherein the intein is between the N- and/or C-terminus or both of the desired protein and the ELP domain;
wherein the codons of the recombinant expression system are optimized for expression in plants and said expression is controlled by optimal promoters;
(b) removing insoluble materials from said extract;
(c) aggregating the fusion proteins by adjusting pH, temperature and salt component, and separating them from the remainder of the extract by microfiltration;
(d) resolubilizing the fusion proteins into an aqueous solution by optimizing pH, temperature and salt component;
(e) repeating steps (c) and (d);
(f) effecting cleavage of the fusion protein by adjusting temperature, pH and providing freeze-thaw treatment; and
(g) separating the desired protein from the tag(s) in a step that comprises aggregating and separating pelleted ELP-intein tags by centrifugation leaving the desired protein in solution;
wherein the ELP domain contains at least 10 ELP units; and
wherein the fusion protein further includes one or more linker sequences between the desired protein and intein and/or intenin and ELP and/or among ELP units.

2. Plants, plant parts or plant cells that contain a nucleic acid which is operable in plant cells to express in said plant cells a fusion protein comprising at either the N-terminus or C-terminus or both of a desired protein, an ELP-intein tag which comprises an elastin-like polypeptide (ELP) domain and an intein splicing element (intein) wherein the intein is between the N- and/or C-terminus or both of the desired protein and the ELP domain,
wherein the ELP domain contains at least 10 ELP units; and
wherein the fusion protein further includes one or more linker sequences between the desired protein and intein and/or intenin and ELP and/or among ELP units, and the codons of the recombinant expression system are optimized for expression in plants.

3. The plants, plant cells or plant parts of claim 2:
(i) wherein the plants, plant parts or plant cells are tobacco and the expression system comprises in the 5' to 3' orientation: a 35S promoter, a sequence encoding ubiquitin, a sequence encoding a phaseolin signal peptide, a sequence encoding a desired protein, a sequence encoding an intein, a linker sequence, a sequence encoding at least 10 ELP repeats and a NOS terminator sequence; or
(ii) wherein the plants, plant parts or plant cells are rice and the expression system comprises in the 5' to 3' orientation a glutelin promoter, a sequence encoding a phaseolin signal peptide, a sequence encoding at least 10 ELP repeats, a linker sequence, a sequence encoding an intein, a sequence encoding a desired protein and a NOS terminator sequence.

4. The plants, plant parts or plant cell of claim 3 wherein in (i) the desired protein is a human granulocyte stimulating factor and in (ii) the desired protein is a *Pandanus* lectin.

5. A method to produce a fusion protein comprising a fusion protein having an ELP- intein tag at the N- and/or C-terminus of a desired protein which method comprises culturing the plants, plant parts or plant cells of claim 4.

6. A recombinant expression system which is a nucleic acid operable in plant cells to express stably in said plant cells a fusion protein comprising at either the N-terminus or C-terminus or both of a desired protein, an ELP-intein tag which tag comprises an elastin-like polypeptide (ELP) domain and an intein splicing element (intein) wherein the intein is between the N- and/or C-terminus or both of the desired protein and the ELP domain,
which nucleic acid comprises a nucleotide sequence encoding said fusion protein operably linked to a promoter that functions in plant cells,
wherein the ELP domain contains at least 10 ELP units,
wherein the fusion protein further includes one or more linker sequences between the desired protein and intein and/or intein and ELP and/or among ELP units and the codons of the recombinant expression system are optimized for expression in plants, and
(i) which comprises in the 5' to 3' orientation: a 35S promoter, a sequence encoding ubiquitin, a sequence encoding a phaseolin signal peptide, a sequence encoding a desired protein, a sequence encoding an intein, a linker sequence, a sequence encoding ELP repeats and a NOS terminator sequence, wherein the desired protein is human granulocyte colony stimulating factor; or
(ii) which comprises in the 5' to 3' orientation a glutelin promoter, a sequence encoding a phaseolin signal peptide, a sequence encoding ELP repeats, a linker sequence, a sequence encoding an intein, a sequence encoding a desired protein and a NOS terminator sequence, wherein the desired protein is *Pandanus* lectin protein.

## Patentansprüche

1. Verfahren zum Erhalt eines gewünschten Proteins aus Pflanzen, Pflanzenteilen oder Pflanzenzellen, wobei das Verfahren folgendes umfasst:
(a) Herstellen bei optimalem pH und Temperatur eines Extrakts aus Proteinen von den Pflanzen, Planzenteilen oder Pflanzenzellen, die das gewünschte Protein stabil als ein Fusionsprotein mit Intein und ELP aus einem solchen optimierten rekombinanten Expressionssystem produzieren, welches das Fusionsprotein herstellt, wobei das Fusionsprotein entweder an dem N-Terminus oder dem C-Terminus eines gewünschten Proteins oder beiden ein ELP-Intein-Tag aufweist, der eine Domäne eines elastinartigen Polypeptids (ELP) und ein Intein-Splicing-Element (Intein) aufweist, wobei das Intein zwischen dem N- und/oder dem C-Terminus des gewünschten Proteins oder beiden und der ELP-Domäne ist;
wobei die Codons von dem rekombinanten Expressionssystem für eine Expression in Pflanzen optimiert sind und die Expression durch optimale Promotoren kontrolliert ist;
(b) Entfernen von unlöslichen Materialien von dem Extrakt;
(c) Aggregieren der Fusionsproteine durch Einstellen von pH, Temperatur und Salzkomponenten, und deren Abtrennen vom Rest des Extraktes durch Mikrofiltration;
(d) Resolubilisieren der Fusionsproteine in eine wässrige Lösung durch optimieren von pH, Temperatur und Salzkomponente;
(e) Wiederholen der Schritte (c) und (d);
(f) Bewirken einer Spaltung des Fusionsproteins durch Einstellen von Temperatur, pH und Bereitstellen einer Gefrier-Auftau-Behandlung; und
(g) Abtrennen des gewünschten Proteins von dem/den Tag(s) in einem Schritt, der das Aggregieren und Abtrennen von pelletierten ELP-Intein-Tag(s) durch eine Zentrifugation umfasst, bei der das gewünschte Protein in Lösung bleibt;
wobei die ELP-Domäne zumindest 10 ELP-Einheiten enthält; und
wobei das Fusionsprotein ferner eine oder mehrere Linkersequenzen zwischen dem gewünschten Protein und Intein und/oder Intein und ELP und/oder inmitten der ELP-Einheiten beinhaltet.

2. Pflanzen, Pflanzenteile oder Pflanzenzellen, die eine Nukleinsäure enthalten, die in Pflanzenzellen operabel ist, um in den Pflanzenzellen ein Fusionsprotein zu exprimieren, das entweder an dem N-Terminus oder dem C-Terminus des gewünschten Proteins oder beiden ein ELP-Intein-Tag aufweist, der eine Domäne eines elastinartigen Polypeptids (ELP) und ein Intein-Splicing-Element (Intein) aufweist, wobei das Intein zwischen dem N- und/oder dem C-Terminus des gewünschten Proteins oder beiden und der ELP-Domäne ist,
wobei die ELP-Domäne zumindest 10 ELP-Einheiten enthält; und
wobei das Fusionsprotein ferner eine oder mehr Linkersequenzen zwischen dem gewünschten Protein und Intein und/oder Intein und ELP und/oder inmitten der ELP-Einheiten beinhaltet, und die Codons des rekombinanten Expressionssystems für die Expression in Pflanzen optimiert sind.

3. Pflanzen, Pflanzenzellen oder Pflanzenteile nach Anspruch 2:
(i) wobei die Pflanzen, Pflanzenteile oder Pflanzenzellen Tabak sind und das Expressionssystem in der 5' nach 3' Orientierung folgendes aufweist: einen 35S-Promotor, eine Sequenz codierend Ubiquitin, eine Sequenz codierend ein Phaseolin-Signalpeptid, eine Sequenz codierend ein gewünschtes Protein, eine Sequenz codierend ein Intein, eine Linkersequenz, eine Sequenz codierend zumindest 10 ELP Wiederholungen und eine NOS-Terminatorsequenz; oder
(ii) wobei die Pflanzen, Pflanzenteile oder Pflanzenzellen Reis sind und das Expressionssystem in der 5' nach 3' Orientierung folgendes aufweist: ein Glutelin-Promotor, eine Sequenz codierend ein Phaseolin-Signalpeptid, eine Sequenz codierend zumindest 10 ELP Wiederholungen, eine Linkersequenz, eine Sequenz codierend ein Intein, eine Sequenz codierend ein gewünschtes Protein und eine NOS-Terminatorsequenz.

4. Pflanzen, Pflanzenteile oder Pflanzenzelle nach Anspruch 3, wobei in (i) das gewünschte Protein ein humaner Granulozyten-stimulierender Faktor ist, und in (ii) das gewünschte Protein ein *Pandanus* Lektin ist.

5. Verfahren zur Herstellung eines Fusionsproteins, das ein Fusionsprotein aufweist, das einen ELP-Intein-Tag an dem N- und/oder dem C-Terminus eines gewünschten Proteins aufweist, wobei das Verfahren das Kultivieren der Pflanzen, Pflanzenteile oder Pflanzenzelle nach Anspruch 4 umfasst.

6. Rekombinantes Expressionssystem, das eine Nukleinsäure ist, die in Pflanzenzellen operabel ist, um in den Pflanzenzellen stabil ein Fusionsprotein zu exprimieren, das entweder an dem N-Terminus oder dem C-Terminus des gewünschten Proteins oder beiden einen ELP-Intein-Tag aufweist, wobei der Tag eine Domäne eines elastinartigen Polypeptids (ELP) und ein Intein-Splicing-Element (Intein) aufweist, wobei das Intein zwischen dem N- und/oder dem C-Terminus des gewünschten Proteins oder beiden und der ELP-Domäne ist,
wobei die Nukleinsäure eine Nukleotidsequenz aufweist, die für das Fusionsprotein codiert und operativ mit einem Promotor verbunden ist, der in Pflanzenzellen funktioniert,
wobei die ELP-Domäne zumindest 10 ELP-Einheiten enthält,
wobei das Fusionsprotein ferner eine oder mehrere Linkersequenzen zwischen dem gewünschten Protein und Intein und/oder Intein und ELP und/oder inmitten der ELP-Einheiten beinhaltet, und die Codons des rekombinanten Expressionssystem für die Expression in Pflanzen optimiert sind, und
(i) welches in der 5' nach 3' Orientierung folgendes aufweist: einen 35S-Promotor, eine Sequenz codierend Ubiquitin, eine Sequenz codierend ein Phaseolin-Signalpeptid, eine Sequenz codierend ein gewünschtes Protein, eine Sequenz codierend ein Intein, eine Linkersequenz, eine Sequenz codierend ELP Wiederholungen und eine NOS-Terminatorsequenz, wobei das gewünschte Protein ein humaner Granulozytenkolonie-stimulierender Faktor ist; oder
(ii) das in 5' nach 3' Orientierung folgendes aufweist: einen Glutelin-Promotor, eine Sequenz codierend ein Phaseolin-Signalpeptid, eine Sequenz codierend ELP-Wiederholungen, eine Linkersequenz, eine Sequenz codierend ein Intein, eine Sequenz codierend ein gewünschtes Protein und eine NOS-Terminatorsequenz, wobei das gewünschte Protein ein *Pandanus* Lektinprotein ist.

## Revendications

1. Procédé pour obtenir une protéine souhaitée à partir de plantes, de parties de plantes ou de cellules végétales, lequel procédé comprend le fait
(a) de préparer, à un pH et à une température optimaux, un extrait de protéines provenant desdites plantes, desdites parties de plantes ou desdites cellules végétales, qui produisent de manière stable ladite protéine souhaitée en tant que protéine de fusion avec une intéine et un ELP à partir d'un système d'expression recombinant optimisé qui produit ladite protéine de fusion, où ladite protéine de fusion comprend au niveau de l'extrémité N-terminale et/ou au niveau de l'extrémité C-terminale d'une protéine souhaitée, une étiquette ELP-intéine qui comprend un domaine de polypeptide de type élastine (ELP) et un élément d'épissage d'intéine (intéine) où l'intéine se trouve entre l'extrémité N et/ou C-terminale de la protéine souhaitée et le domaine ELP ;
dans lequel les codons du système d'expression recombinant sont optimisés pour l'expression dans des plantes et ladite expression est contrôlée par des promoteurs optimaux ;
(b) de retirer les matières insolubles dudit extrait ;
(c) d'agréger les protéines de fusion par réglage du pH, de la température et du composant sel, et de les séparer du reste de l'extrait par microfiltration ;
(d) de resolubiliser les protéines de fusion dans une solution aqueuse en optimisant le pH, la température et le composant sel ;
(e) de répéter les étapes (c) et (d)
(f) d'effectuer le clivage de la protéine de fusion en réglant la température, le pH et en fournissant un traitement de congélation-décongélation ; et
(g) de séparer la protéine souhaitée de l'étiquette/des étiquettes dans une étape qui comprend l'agrégation et la séparation d'étiquettes ELP-intéine granulées par centrifugation en libérant la protéine souhaitée dans la solution ;
dans lequel le domaine ELP contient au moins 10 unités d'ELP ; et
dans lequel la protéine de fusion comporte en outre une ou plusieurs séquence(s) de lieur entre la protéine souhaitée et l'intéine et/ou l'intéine et l'ELP et/ou entre les unités d'ELP.

2. Plantes, parties de plantes ou cellules végétales qui contiennent un acide nucléique qui peut fonctionner dans des cellules végétales pour exprimer dans lesdites cellules végétales une protéine de fusion comprenant au niveau de l'extrémité N-terminale et/ou au niveau de l'extrémité C-terminale d'une protéine souhaitée, une étiquette ELP-intéine qui comprend un domaine de polypeptide de type élastine (ELP) et un élément d'épissage d'intéine (intéine) où l'intéine se trouve entre l'extrémité N et/ou C-terminale de la protéine souhaitée et le domaine ELP,
où le domaine ELP contient au moins 10 unités d'ELP ; et
où la protéine de fusion comporte en outre une ou plusieurs séquence(s) de lieur entre la protéine souhaitée et l'intéine et/ou l'intéine et l'ELP et/ou entre les unités d'ELP, et les codons du système d'expression recombinant sont optimisés pour l'expression dans des plantes.

3. Plantes, cellules végétales ou parties de plantes de la revendication 2 :
(i) dans lesquelles les plantes, les parties de plantes ou les cellules végétales sont le tabac et le système d'expression comprend dans l'orientation 5' à 3' : un promoteur 35S, une séquence codant pour l'ubiquitine, une séquence codant pour un peptide signal de phaséoline, une séquence codant pour une protéine souhaitée, une séquence codant pour une intéine, une séquence de lieur, une séquence codant pour au moins 10 répétitions d'ELP et une séquence de terminaison NOS ; ou
(ii) dans lesquelles les plantes, les parties de plantes ou les cellules végétales sont le riz et le système d'expression comprend, dans l'orientation 5' à 3', un promoteur de glutéline, une séquence codant pour un peptide signal de phaséoline, une séquence codant pour au moins 10 répétitions d'ELP, une séquence de lieur, une séquence codant pour une intéine, une séquence codant pour une protéine souhaitée et une séquence de terminaison NOS.

4. Plantes, parties de plantes ou cellules végétales de la revendication 3, dans lesquelles, dans (i), la protéine souhaitée est un facteur de stimulation de granulocytes humain et dans (ii) la protéine souhaitée est une lectine de *Pandanus.*

5. Procédé pour produire une protéine de fusion comprenant une protéine de fusion ayant une étiquette ELP- intéine au niveau de l'extrémité N et/ou C terminale d'une protéine souhaitée, lequel procédé comprend la mise en culture des plantes, des parties de plantes ou des cellules végétales de la revendication 4.

6. Système d'expression recombinant qui est un acide nucléique pouvant fonctionner dans des cellules végétales pour exprimer de manière stable dans lesdites cellules végétales, une protéine de fusion comprenant au niveau de l'extrémité N-terminale et/ou au niveau de l'extrémité C-terminale d'une protéine souhaitée, une étiquette ELP-intéine, laquelle étiquette comprend un domaine de polypeptide de type élastine (ELP) et un élément d'épissage d'intéine (intéine) où l'intéine se trouve entre l'extrémité N et/ou C-terminale de la protéine souhaitée et le domaine ELP,
lequel acide nucléique comprend une séquence nucléotidique codant pour ladite protéine de fusion liée de manière fonctionnelle à un promoteur qui fonctionne dans des cellules végétales,
dans lequel le domaine ELP contient au moins 10 unités d'ELP,
dans lequel la protéine de fusion comporte en outre une ou plusieurs séquence(s) de lieur entre la protéine souhaitée et l'intéine et/ou l'intéine et l'ELP et/ou entre les unités d'ELP et les codons du système d'expression recombinant sont optimisés pour l'expression dans des plantes, et
(i) qui comprend dans l'orientation 5' à 3' : un promoteur 35S, une séquence codant pour l'ubiquitine, une séquence codant pour un peptide signal de phaséoline, une séquence codant pour une protéine souhaitée, une séquence codant pour une intéine, une séquence de lieur, une séquence codant pour des répétitions d'ELP et une séquence de terminaison NOS, où la protéine souhaitée est un facteur de stimulation de colonies de granulocytes humain ; ou
(ii) qui comprend, dans l'orientation 5' à 3', un promoteur de glutéline, une séquence codant pour un peptide signal de phaséoline, une séquence codant pour des répétitions d'ELP, une séquence de lieur, une séquence codant pour une intéine, une séquence codant pour une protéine souhaitée et une séquence de terminaison NOS, où la protéine souhaitée est une protéine lectine de *Pandanus.*
